# EUROPEAN PATENT SPECIFICATION

(11) **EP 4 327 836 B1**
(45) Date of publication and mention of the grant of the patent: **08.10.2025**
(21) Application number: 23193436.5
(22) Date of filing: 25.08.2023
(51) Int. Cl.: A61L 2/20, A61L 2/24, A61L 9/12, A61L 9/14

(54) **AIRBORNE SURFACE DISINFECTION BY CHLORINE DIOXIDE GAS USING ULTRASONIC-ASSISTED FUMIGATION**
DESINFEKTION VON LUFTOBERFLÄCHEN DURCH CHLORDIOXIDGAS MITTELS ULTRASCHALLGESTÜTZTER BEGASUNG
DÉSINFECTION DE SURFACE EN SUSPENSION DANS L'AIR PAR DU DIOXYDE DE CHLORE GAZEUX À L'AIDE D'UNE FUMIGATION ASSISTÉE PAR ULTRASONS

(30) Priority: 26.08.2022 BE 202205680
(43) Date of publication of application: 28.02.2024
(73) Proprietor: Konax BV, 8870 Izegem (BE)
(72) Inventor: Demyttenaere, Karel, 8700 Izegem (BE); Goudezeune, Stefaan, 8700 Izegem (BE)
(74) Representative: Brantsandpatents bv

(56) References cited:
- CN-A- 101 366 965
- CN-A- 113 499 464
- CN-U- 212 619 082
- JP-A- 2015 155 363

## Description

### FIELD OF THE INVENTION

The present invention relates to a method and a device for disinfecting and/or sterilizing a room and/or apparatus placed in a room by means of chlorine dioxide gas.

### BACKGROUND

Chlorine dioxide is a powerful and broad-spectrum disinfectant that can be used either as a gas or a liquid. Given its low boiling point (11 °C), when it is dissolved in water, it is partially distributed between the liquid and the head phase under normal atmospheric conditions. Chlorine dioxide can easily penetrate the cell membrane of microorganisms and prevent protein production and hence killing the microorganism.

Thanks to its remarkable efficacy against different types of microorganisms such as bacteria, viruses, spores, fungi, chlorine dioxide has been used for disinfection of different types of water such as drinking water, process water, cooling power, agriculture, horticulture, etc. Disinfection of surfaces in enclosed spaces such as hospitals, microbiological labs and microbial-sensitive areas in pharmaceutical and food companies is another application where chlorine dioxide can be used as an effective disinfectant. Chlorine dioxide is used as a biocide or oxidant, for example for water treatment or odor control. Chlorine dioxide has a relatively low oxidation potential compared to other oxidants but nevertheless has a high oxidation capacity. Moreover, when used as a disinfectant, it forms less by-products compared to chlorine.

JP 2015155363 describes a chlorine dioxide gas generator and method which reduces microorganisms by mixing of acidic mist comprising chlorine dioxide gas to be released at a low humidity. The chlorine dioxide gas generator for disinfecting a room with chlorine dioxide gas by creating an acid mist in a reaction cell.

CN 113499464 describes a method and device for disinfecting a nut packaging workshop. The chlorine dioxide gas is released into a packaging function area in two stages and at two concentrations of chlorine dioxide gas, low in a manned environment and high in an unmanned environment. The device therefore comprises a metering pump and an in-line sensor for measuring the concentration of chlorine dioxide gas.

CN 101366965 discloses an ultrasonic vibrator using an ultrasound oscillator made of stainless steel. This device is used in an aerosol machine to release chlorine dioxide disinfection liquid to form micro-sized droplets of chlorine dioxide, which are effective for air sterilization without wetting the environment.

CN 212619082 discloses an ultrasonic atomization degassing unit used for indoor environment disinfection. The device comprises a reaction cell for mixing chlorine dioxide solution and activation liquid, which is coupled to an ultrasonic atomization generator. This generator produces a mist that is discharged in to a central air conditioning or ventilation system.

The chlorine dioxide gas can be generated by a wide range of methods. However, the majority of these methods suffer from high corrosive nature, complicated and expensive instrumentation as well as long preparation times. Therefore, the possible use of chlorine dioxide gas technology is largely limited, and mainly for the applications in which chlorine dioxide is extremely useful. Thus, there is a need for low cost, quick and less corrosive techniques by which the chlorine dioxide can be released into the room to be disinfected without reducing the efficiency. The present invention aims to offer a solution for one or more of the said problems.

### SUMMARY OF THE INVENTION

In a first aspect, the invention serves to provide a solution to one or more of above-mentioned disadvantages. To this end, the present invention relates to a method for disinfecting a room with chlorine dioxide gas according to claim 1. According to this method for airborne disinfection of surfaces by chlorine dioxide gas, the chlorine dioxide gas is directly released to space by ultrasonic vibration from a concentrated chlorine dioxide solution at a predetermined and constant humidity. The desired humidity is determined based on the initial humidity of the room, the corrosion sensitivity of the equipment present in the room and the desired disinfection level. The exposure time is determined based on the active concentration of chlorine dioxide gas present in the room, since the moment the humidity of the room reaches the desired humidity, and the desired disinfection level.

The current invention relates to an improved method of airborne surface disinfection wherein an aqueous solution of chlorine dioxide is prepared at the point-of-use and the chlorine dioxide gas is released into the room by applying ultrasonic vibration in the prepared solution. The method describes an effective, easy-to-operate and low-cost technique for disinfection of critical environments such as biological labs, clean rooms, hospitals, and especially places where corrosion-sensitive objects or tools are required to be disinfected. Preferred embodiments of the method are shown in any of the claims 2 to 12.

In a second aspect, the present invention relates to a device according to claim 13. More particular, the device as described herein aims to control the RH in real time during disinfection with chlorine dioxide gas. A RH sensor, suitable to measure the relative humidity in the room, a dehumidifier suitable to decrease the RH in case the RH is above the desired RH and software to precisely control the fumigation process are provided. A RH above the desired level can cause corrosion of the items in the room. A RH lower than the desired level reduces the disinfection efficiency unnecessarily. Therefore, proper control of this parameter is desired.

### DETAILED DESCRIPTION OF THE INVENTION

There are different techniques that can be used for air and surface disinfection including but not limited to ultraviolet (UV) radiation, vaporized hydrogen peroxide (VHP) or hydrogen peroxide nebulizer. However, these techniques suffer from some drawbacks that limit their application. For example, in the UV disinfection systems, the method efficiency strongly depends on the exposure time and distance from the UV source. So, multiple lamps and long exposure times are often required to ensure every corner and surface is exposed to UV light and is disinfected. To achieve the maximum efficiency, it is very important that the place that is supposed to be disinfected is as clean as possible. Thick layers of dirt or liquids over floors, walls or equipment prevent the UV light from reaching the surfaces efficiently. As a result, it is recommended that UV disinfection is combined with other techniques. Disinfection systems based on hydrogen peroxide like VHP is another technique that has been widely applied for airborne disinfection. However, as the name implies, hydrogen peroxide is used as a vapor which reduces its diffusion and penetration capability markedly. Besides, hydrogen peroxide is very corrosive which limits its application.

Gas fumigation with formaldehyde or ethylene oxide is another approach which is very limited, as these compounds are very toxic, flammable, explosive, and carcinogenic in nature, and have the potential to cause severe health and environmental problems.

On the other hand, disinfection by chlorine dioxide gas has several distinctive advantages that makes it attractive for airborne surface disinfection. In the first place, it is more effective than the UV system and VHP. Because chlorine dioxide is a gas that can diffuse equally everywhere. So, the gas concentration will be the same at every corner resulting in a homogenous disinfection at short exposure times. Secondly, the penetration of chlorine dioxide gas is much higher than UV and VHP as it's a gas. Besides, it has a lower oxidation power which is very useful for the environments with corrosion-sensitive objects. Last but not least, chlorine dioxide has no carcinogenic effect and leaves no residue on the treated surfaces.

The most frequently used disinfection technique using chlorine dioxide gas is based on the direct reaction of chlorine gas in a stream of nitrogen gas with sodium chlorite. However, this technique suffers from serious problems. The method involves bulky equipment such as a chlorine gas cylinder and big monitoring systems which makes the operation very laborious. A lot of tubing is required to carry out the generated gas to the room. The tubing and connections should be fully airtight. Otherwise, the gas is very likely to enter the monitoring room where the operator is working. The room needs to be equipped with the suitable built-in openings so that the gas can be sent to the room, and the treated air can be sampled for the exposure time calculation. Since chlorine gas is used as the starting material, there is always a high risk of corrosion not only for the valve and connections but also for the objects in the room. The chemical reactions involved in corrosion of metal objects with chlorine gas are provided below. In the first step, the metals are oxidized by chlorine and hydrochloric acids, forming metal chlorides. See reactions (1-3).

M(s) + Cl₂(g) → MCl₂(s) (1)

M(s) + 2HCl(g) → MCl₂(g) + H₂(g) (2)

MCl₂(s) → MCl₂(g) (3)

In the second step, the metal chlorides will get oxidized by the air leading to formation of solid metal oxides that create a layer of rust over the surface of metallic objects. See reactions (4-5).

3MCl₂(g) + 2O₂(g) → M₃O₄(s) + 3Cl₂(g) (4)

2MCl₂(g) + (3/2)O₂(g) → M₂O₃(s) + 2Cl₂(g) (5)

The corrosion reactions can be further catalyzed by the moisture. So, the humidity control is very important for any airborne surface disinfection.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

"A", "an", and "the" as used herein refers to both singular and plural referents unless the context clearly dictates otherwise. By way of example, "a compartment" refers to one or more than one compartment.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

Exposure is defined as time (minutes) x concentration (mg/L). Exposure time is expressed in minutes or hours.

A room can be any size. A container, storage box or entire buildings can also be considered rooms. The skilled person knows that the disinfecting method can be applied to small and big spaces. A room is herein used as a synonym for a space to be disinfected.

The term "disinfecting" is used as a synonym for the term "decontaminating" or "purifying" and refers to at least partially destroying one of more types of kind of plagues, pathogens or germs of a disease. Preferably, at least 99% of said pathogens or germs of a disease are destroyed, more preferably at least 99.99% and most preferably 99.999%. The term "kind of plague" is meant as a synonym of the term "noxious organism" and refers to each organism that is present unwantedly or that has a negative effect on a human, animal, plant and/or the environment. Examples of kinds of plagues are weed, micro-organisms, pathogens, fungi, larvae, insects, parasites, nematodes, algae, mites, rodents, bacteria, viruses, etc. The term "pathogen" or "pathogens" is used as a synonym for the term "pathogenic organism" and refers to several bacteria, viruses, fungi, yeasts and protozoa that can cause disease and/or death of a human, animal, plant or other biological organism. Pathogenic spores are spores that are produced by a pathogen. Specific examples of pathogens that produce spores, include, but are not limited to, members of the genera Bacillus, Clostridium, Desulfotomaculans, Sporolactobacillus, and Sporpsarcina, members of the Phylum Apicomplexa (such as Plasmodium falciparum and Cryptosporidium parvum), and phytopathogenic fungi. In the context of the present invention, the term "disinfecting" is also meant as "sterilizing". When sterilizing, a higher degree of killing of pathogens is obtained compared to disinfecting: degree 'log 6' when sterilizing compared to degree 'log 5' when disinfecting. The sterilization of a room is mainly carried out in applications where a high degree of hygiene is required, such as for example in rooms for food treatment or production, in medical rooms such as for example in hospitals, as well as in veterinary applications. Disinfecting comprises a period in which the concentration of disinfecting agent increases, a period in which the concentration of disinfecting agent is maintained, and a period in which the concentration of disinfecting agent is reduced. These 3 periods are herein called increasing period, constant period and reducing period, respectively. The defined exposure time is the duration of the constant period. The speed at which the concentration of the disinfecting agent increases during the increasing period is variable.

The term "chlorine dioxide" or "ClO₂" refers to a molecule indicated with the CAS number 10049-04-4 and appears as a gas at standard pressure and temperature. Chlorine dioxide has a greenish, yellow color with a characteristic odor similar to chlorine and is a particularly efficient biocide that kill pathogens, such as bacteria, viruses and parasites, fast and efficiently. Chlorine dioxide gas molecules can also kill atomized germs of a disease and can also spread through tears and cracks in an article or a building or a room and thus reach each surface that is possibly contaminated with a germ of a disease. Chlorine dioxide is very well water-soluble, but compared to chlorine, it does not react with water. It appears in an aqueous solution as a dissolved gas. Chlorine dioxide is recognized as oxidative, possibly explosive, corrosive, toxic and environmentally dangerous. The boiling point at standard pressure of chlorine dioxide is 11°C.

The term "sodium chlorite" refers to a chemical molecule with the gross formula NaClO₂ and is indicated with the CAS number 7758-19-2. The term "sodium bisulphate" refers to a chemical molecule with the gross formula NaHSO4 and is indicated with the CAS number 7681-38-1.

The term "reaction cell" is meant as a reaction vessel in which solid, liquid and/or gaseous agents can be loaded and discharges. Preferably, said reaction cell contains an aqueous solution in which chemical reactions can take place.

The term desired process humidity is meant as the relative humidity at which the disinfection should be performed. This is determined based on the desired disinfection degree and corrosion sensitivity of the equipment.

The term desired initial humidity is meant as the relative humidity at which the chlorine dioxide gas generation (sonication) should start. In order to find this value, the humidity margin caused by the sonication should be deducted from desired process humidity.

In a first aspect, the invention provides/relates to a method for disinfecting a room with chlorine dioxide gas, comprising the steps:
measuring the humidity of the air in the room;
generating chlorine dioxide from a liquid in a reaction cell;
releasing said chlorine dioxide from said liquid;
transferring the released chlorine dioxide to the room;
measuring the concentration chlorine dioxide gas in the room; and
maintaining a constant chlorine dioxide concentration in the air in the room for a defined exposure time.

In an embodiment, the invention relates to a method for disinfecting a room with chlorine dioxide gas, comprising the steps:
measuring the humidity of the air in the room;
finding the desired initial humidity of the room;
finding the desired process humidity for disinfection of the room
reducing the humidity of the room if it is above the desired initial humidity;
generating chlorine dioxide from a liquid in a reaction cell;
releasing said chlorine dioxide gas from said liquid to the room;
transferring the released chlorine dioxide to the room;
measuring the humidity of the room in real time;
stopping the gas generation at the desired process humidity;
measuring the concentration chlorine dioxide gas in the room; and
maintaining a constant chlorine dioxide concentration in the air in the room for a defined exposure time.

In a further embodiment, the relative humidity (RH) in the room is kept constant during the defined exposure time at a predetermined RH between 20% and 80%, preferably between 30% and 80%, more preferably between 50% and 80%, even more preferably between 65% and 75%.

The method describes an effective, quick, easy-to-operate and low-cost technique for disinfection of critical environments such as biological labs, clean rooms, hospitals, and especially places where corrosion-sensitive objects or tools are required to be disinfected.

The present invention provides an improved disinfection system using chlorine dioxide gas. The gas injection will continue until the desired relative humidity (RH) is achieved. To control the gas injection, a relative humidity sensor and an in-line chlorine dioxide gas sensor are used to measure the RH and chlorine dioxide concentration in a real-time fashion. A dehumidifier will be used if the RH of the room exceeds the desired RH. A software will be developed and used for precise control of the fumigation process. The exposure time is determined based on the actual gas concentration from the moment the desired RH is achieved. This results in several unexpected advantages over the existing gas systems. Firstly, the generation of chlorine dioxide gas is completely chlorine-free. So, there is little concern about corrosion of equipment and tools by residual chlorine. Secondly, higher efficiencies are expected in shorter disinfection times due to performing the fumigation at an optimum RH at which at least 99 % of microorganisms are killed. Third, the operational RH is determined based on the type of equipment available in the room and is fully controlled during the procedure to minimize the corrosion.

Finally, the operation is very safe as the gas is created in a very safe condition with minimum safety risk for the operator and users. The chemicals consumption and operational costs are quite low, and the disinfection process is very simple and easy-to-operate. There is no need for multiple and long tubings as the gas is generated from concentrated chlorine dioxide solutions placed inside the room. The monitoring system will also be placed inside the room. So, everything will be executed inside the room while the operator stands outside.

In an embodiment, measuring the humidity of the air in the room is conducted using a sling psychrometer. A sling psychrometer yields an accurate result and is easy to use. In an embodiment, the generating chlorine dioxide from a liquid in a reaction cell occurs after a reaction between a product comprising Cl and an oxidant, preferably reacting of sodium chlorite with chlorine. In an embodiment, the generating chlorine dioxide from a liquid in a reaction cell occurs after a reaction between potassium chlorate with oxalic acid. In an embodiment, the releasing said chlorine dioxide from said liquid occurs using a vibrating plate. In an embodiment, the releasing said chlorine dioxide from said liquid occurs by flushing the liquid with a gas. In an embodiment, the transferring of the released chlorine dioxide to the room is achieved using a pump. In an embodiment, the measuring the concentration of chlorine dioxide gas in the room is conducted using a spectrophotometer. A spectrophotometer yields an accurate result and is easy to use. A spectrophotometer is suitable to report results constantly. In an embodiment, a constant chlorine dioxide concentration in the air in the room is maintained by measuring the chlorine dioxide concentration in the room and adjusting the vibration speed of the vibrating plate in the liquid. In an embodiment, a constant chlorine dioxide concentration in the air in the room is maintained by measuring the chlorine dioxide concentration in the room and adjusting the concentration of chlorine dioxide in the liquid.

In an embodiment, the predetermined RH is dependent on the sensitivity of the room and the items in the room which should be disinfected. If the room contains very sensitive items, the predetermined RH during disinfection is 20-40%, preferably 30-40%. If the room does not contain any items sensitive to corrosion, the predetermined RH during disinfection is set at 50-80%, preferably 65-75%. If the room contains for example stainless steel equipment, the predetermined RH during disinfection is set at 40-75%, preferably 50-75%, more preferably between 65 and 75%. In an embodiment, the predetermined RH is between 30 and 50%. In another embodiment, the predetermined RH is between 40 and 60%.

In a preferred embodiment of the invention, the method further comprises a step wherein the RH in the room is adjusted based on the desired RH and the RH margin caused by sonication of chlorine dioxide solution between 20% and 50%, preferably between 40% and 50%, prior to the transferring of the released chlorine dioxide gas to the room. In an embodiment, the RH is measured in the room and a dehumidifier reduces the RH to below 50%. The dehumidifier will be only required when the actual RH is higher than the required RH to start the fumigation. When the chlorine dioxide gas is released into the room, small amounts of water droplets or water vapor also enter the room. The RH steadily increases. In an embodiment, the transferring of the released chlorine dioxide to the room is stopped when the predetermined RH is reached. If the concentration of chlorine dioxide in the room is not sufficient, a dehumidifier reduces the RH to below 50%. Then, the sonication starts again to generate the required chlorine dioxide gas in the room. The Sonication will be stopped when the RH of the room reaches the desired RH. The gas concentration is measured in line to calculate the exposure time. The system will be maintained at constant RH and concentration for the calculated exposure time.

In an embodiment, the proposed method uses a liquid solution containing 3000-10 000 mg/L of chlorine dioxide, preferably 4000-8000 mg/L. The concentration is depending on the size of the area that should be decontaminated. In an embodiment, the generation of the chlorine dioxide gas is conducted following the following 3 steps:
1) A liquid solution containing 3000-10 000 mg/L of chlorine dioxide is generated in-situ and placed in a reaction cell, preferably the liquid solution contains 4000-8000 mg/L of chlorine dioxide, more preferably 5000-7000 mg/L. The solution preparation is not decisive and different techniques can be considered. In an embodiment, the solution is prepared via sodium chlorite electrolysis, oxidation of sodium chlorite by hydrochloric acid, sulfuric acid or sodium bisulfate, reduction of sodium chlorate, etc.
2) The reaction cell is equipped with a vibrating plate that generates ultrasonic vibrations. As a result, the hydrated chlorine dioxide comes out of the solution as a gas and diffuses into the air where the generator is placed.
3) After exposure, the solution is discharged into a bottle with a neutralizing agent such as sodium thiosulfate.

In a preferred embodiment of the invention, the defined exposure time is determined based on the required level of disinfection and the concentration of chlorine dioxide in the room.

The disinfection protocol determines the exposure of the space or object to be disinfected by the disinfectant. The exposure is expressed in duration (time) x concentration (ppm). The exposure can vary depending on the micro-organism to be killed, the extent to which the disinfection should be performed, the concentration of the disinfectant, and the desired disinfection time. In an embodiment, the concentration of chlorine dioxide during the constant period of the disinfection is 400 ppm ± 15%, preferably 400 ppm ± 10%, more preferably 400 ± 5%. In an embodiment, the concentration of chlorine dioxide during the constant period of the disinfection is 360 ppm ± 15%, preferably 360 ppm ± 10%, more preferably 360 ± 5%. In an embodiment, the concentration of chlorine dioxide during the constant period of the disinfection is 300 ppm ± 15%, preferably 300 ppm ± 10%, more preferably 300 ± 5%.

Environmental conditions such as temperature and humidity can impact on the effectiveness of the protocol. However, unlike Vaporized Hydrogen Peroxide (VHP) technique in which the hydrogen peroxide concentration is dictated by the humidity of the environment, the humidity has little impact on the concentration of active substance in the proposed technique as it utilizes chlorine dioxide gas. The gas distribution in the air is basically independent of the humidity. On the other hand, the corrosive effect of humidity will remain the same in both techniques. So, a precise humidity control is desired to achieve effective disinfection with Min. corrosion. Humidity has no impact on the concentration of chlorine dioxide in the air, unlike hydrogen peroxide. Humidity has an impact on the resistance of the microorganisms. A higher humidity increases the impact on the cell wall. A higher humidity also increases the corrosive pressure on objects in the room. A fine balance should be sought.

In a preferred embodiment of the invention, the liquid is neutralized after the defined exposure time. The term "neutralize" refers to the chemical neutralization of a chemical agent, preferably by means of chemisorption, physisorption and/or decomposition. As a result of the neutralization, the concentration of chlorine dioxide in said gas stream is substantially reduced. Said chlorine dioxide can for example be neutralized by means of, preferably intensive, contact with an aqueous solution comprising 10% by weight of NaOH. Said chlorine dioxide can for example be neutralized by means of, preferably intensive, contact with an aqueous solution comprising 10% by weight of sodium thiosulfate hydrate. In a preferred embodiment of the invention, at the end of the defined exposure time, the liquid is mixed with sodium thiosulfate. Preferably, said chlorine dioxide is neutralized in a gas washer. In an alternative or preferably additional embodiment, a filter with a fixed chlorine dioxide adsorbent, preferably active coal, is used for adsorbing remaining chlorine dioxide in the disinfected room. Preferably, said filter is appropriate for treating an air volume 15 times the volume of the disinfected room. This offers the advantage that the room to disinfect can be disposed of possibly noxious chlorine dioxide. In an embodiment, the chlorine dioxide in the room is neutralized. This can for example be realized by means of ventilation or repeated ventilation of the treated room. Alternatively, the chlorine dioxide gas can be neutralized. Preferably, the neutralization in said room lasts less than 2 hours, more preferably less than 1 hour, more preferably between 5 minutes and 30 minutes and most preferably 5 minutes, 10 minutes, 15 minutes, 20 minutes, 25 minutes or 30 minutes, or any value between these values.

In a preferred embodiment of the invention, the concentration of chlorine dioxide gas in the room is continuously measured by an in-line sensor. In a preferred embodiment of the invention, the concentration of chlorine dioxide gas in the room is measured every minute by an in-line sensor. To control the process properly, the concentration of chlorine dioxide gas in the room should be known. Preferably the measurement is conducted using a spectrophotometer. In an embodiment, the defined exposure time depends on the concentration of chlorine dioxide gas measured in the room with a spectrophotometer. In an embodiment, the defined exposure time depends on the concentration of chlorine dioxide gas measured in the room with a spectrophotometer and the RH measured with a RH sensor when the transferring of the released chlorine dioxide to the room starts.

In an embodiment, the spectrophotometer comprises: a light emitting diode; a photoreceptor; a sample cell, partially defined by at least two substantially parallel windows made of a material transparent to the light emitted by the at least one light emitting diode and accommodating a sample fluid, wherein the sample cell is located between the at least one light emitting diode and the photoreceptor, such that light produced by the at least one light emitting diode passes through the sample cell in a direction substantially perpendicular to the at least two substantially parallel windows, and impinges on the photoreceptor. In a further embodiment, the at least two windows are made of quartz. In a further embodiment, one of the light emitting diodes produces light in the wavelength range of about 400 to 750 nanometers.

In an embodiment, the liquid comprises 200-50 000 mg/L of chlorine dioxide, preferably 1000-20 000 mg/L. In a preferred embodiment of the invention, the liquid comprises 3000-10 000 mg/L of chlorine dioxide, preferably 4000-8000 mg/L. in an embodiment, the concentration chlorine dioxide in the liquid depends on the volume of the room to be treated. If the room is larger, a higher concentration should be used. This way, the disinfection method can be conducted faster for larger rooms.

In a preferred embodiment of the invention, the method comprises the step of dehumidifying the room when the RH exceeds a target value. In an embodiment, , the method comprises the step of: 1) measuring the RH in the room; 2) dehumidifying the room when the RH exceeds a first target value, preferably the first target value is between 20 and 50% RH, more preferably between 40 and 50%; 3) the chlorine dioxide is transferred to the room while the RH is measured at least every minute; 4) the transferring is stopped and the room is again de-humified if the concentration of chlorine dioxide gas is not within the desired range. Steps 3) and 4) are repeated until the concentration of chlorine dioxide in the room is between 200 and 500 mg/L, preferably between 300 and 420 ppm; 5) determining the defined exposure time; 6) maintaining a constant concentration of chlorine dioxide and RH in the room for the defined exposure time; 7) neutralizing the chlorine dioxide in the liquid and in the room.

In a preferred embodiment of the invention, the liquid obtained by mixing aqueous solutions of sodium chlorite and sodium bisulfate, preferably the liquid is prepared using concentrated sodium chlorite (25% w/w) and sodium hydrogen sulfate 20% (w/v). In an embodiment, the liquid obtained by mixing aqueous solutions of sodium chlorite and sodium bisulfate, preferably the liquid is prepared using diluted sodium chlorite (5% w/w) and sodium hydrogen sulfate 5% (w/v).

In an embodiment of the invention, during the defined exposure time, the concentration of chlorine dioxide in the room is between 50 and 5000 ppm, preferably between 200 and 750 ppm. In a preferred embodiment of the invention, during the defined exposure time, the concentration of chlorine dioxide in the room is between 300 and 420 ppm, preferably between 340 and 380 ppm.

According to the invention, chlorine dioxide gas is generated from said liquid by an ultrasonic vibrator. The chlorine dioxide that exists as a dissolved gas inside the watery solution is released into the space by applying an ultrasonic vibration. In fact, during sonication, bubble formation and subsequent implosion result in extremely high pressure, facilitating release of chlorine dioxide gas. So, in this technique the chlorine dioxide is injected to the space in a gas form. The ultrasonic vibrator is configured to release chlorine dioxide from the liquid in an efficient way. The required energy is lower, and the purity is higher compared to alternative techniques.

In a preferred embodiment of the invention, the defined exposure time is determined based on the relative humidity (RH) in the room once the concentration of chlorine dioxide in the room is between 300 and 420 ppm.

In a preferred embodiment of the invention, 60-100% of the chlorine dioxide is released as a gas from said liquid and 0-40% of the chlorine dioxide is released as droplets with a particle diameter below 40 µm from said liquid. In an embodiment of the invention, 80-100% of the chlorine dioxide is released as a gas from said liquid and 0-20% of the chlorine dioxide is released as droplets with a particle diameter below 30 µm from said liquid. In an embodiment of the invention, 95-100% of the chlorine dioxide is released as a gas from said liquid and 0-5% of the chlorine dioxide is released as droplets with a particle diameter below 20 µm from said liquid.

In an embodiment, said room is partially or completely closed off prior to the disinfection. This offers the advantage that no or only a limited amount of chlorine dioxide gas can leak into the environment, as a result of which the actual concentration of chlorine dioxide remains maximal in the space to be disinfected, and that there is no risk of undesired contact between said chlorine dioxide and adjacent or adjoining spaces and the equipment present therein. Avoiding or at least significantly reducing chlorine dioxide gas leaks to adjacent spaces contributes to the safety of both professional operators and persons in the vicinity of the space to be disinfected. Preferably, said space is sealed by means of a substantially gas-impermeable closure. A sealed space such as a sealed article, sealed space, or a sealed building is to be understood as an environment wherein substantially all fluid conduits with the environment are or have been sealed, for example, by means of plastic covers or other sheets, tape, insulation, sealing or combinations thereof, and wherein preferably said covers are gas impermeable. In a further embodiment, said sealed space is formed by means of a 'glove bag', a 'gas bag', an 'air bag' or an 'atmosbag'. Said sealed space is, nevertheless, preferably provided with one or more in- and/or outlets which allow that specific agents can be moved into and/or out of the sealed space.

In a second aspect, the invention relates to a device for disinfecting a room with chlorine dioxide, comprising:
a RH sensor suitable to measure the relative humidity in the room;
a dehumidifier;
a software with suitable hardware to control the fumigation process
a reaction cell for formulating and/or storing chlorine dioxide gas; and
a dispersing unit in fluidic connection with said reaction cell suitable for dispersing the chlorine dioxide gas from said reaction cell in the room.

The device as described herein aims to control the RH during disinfection with chlorine dioxide. A RH sensor, suitable to measure the relative humidity in the room, and a dehumidifier are provided. A RH above the desired level can cause corrosion of the items in the room. A RH lower than the desired level reduces the disinfection efficiency unnecessarily. Therefore, proper control of this parameter is desired.

The inventors have unexpectedly observed that although the RH has no significant effect on the concentration of chlorine dioxide in the air, the RH influences the corrosion of items exposed to chlorine dioxide. The device measures and evaluates the RH. When the RH is too high, the dehumidifier can reduce the RH. This offers the advantage that a disinfection method is possible without damaging the items in the room. Furthermore, the disinfection can be conducted at RH close to the target value.

According to the invention, the device further comprises:
an in-line sensor suitable to measure the chlorine dioxide gas concentration;
an operation panel which is formed of an input portion to input a sterilization condition, and a display portion to display a sterilization status;
an ultrasonic vibrator; and
an electronic control processor, coupled with the in-line sensor and the operation panel, which is configured to control the dehumidifier and the ultrasonic vibrator.

The ultrasonic vibrator is configured to release chlorine dioxide from the liquid in an efficient way. The required energy is lower, and the purity is higher compared to alternative techniques.

In an embodiment, the electronic control processor converts a sterilization condition set on the operation panel and a concentration measurement value of the chlorine dioxide received from the in-line detecting sensor into an electrical signal and display the converted electrical signal and is configured to control the concentration of the gaseous chlorine dioxide. In an embodiment, the electronic control processor is configured to control the release of chlorine dioxide from the reaction cell to the room. In an embodiment, the electronic control processor is configured to control a door locking device for the door of the room to be closed if gaseous chlorine dioxide remains in the reaction cell. In an embodiment, the electronic control processor is configured to control a door locking device for the door of the room to be closed until after the sterilization is completed. In an embodiment, the electronic control processor is configured to display values and graphs generated during the sterilization procedure, on the operation panel, and the sterilization history is printed using the printer, wherein the above control operations are automatically carried out by a control of the electronic control part.

It is clear for the skilled person that the method described herein can be conducted using the device described in the second aspect.

### EXAMPLES

The present invention will now be further exemplified with reference to the following examples. The present invention is in no way limited to the given examples.

### Example 1. Disinfection of a biological safety cabinet

### Step 1. Preparation of chlorine dioxide solution

A solution containing 5000 mg/L of chlorine dioxide is generated from in-situ reaction between concentrated sodium chlorite (25% w/w) and sodium hydrogen sulphate 20% (w/v).

### Step 2. Conditioning of the room

The relative humidity (RH) of the room is measured before the sonication starts. The optimal RH and chlorine dioxide concentration are determined based on customer requirements, formulas, and the disinfection provider experience. In this example the desired RH is 65% and the required concentration is 360 ppm of chlorine dioxide. The RH will increase by 15% when creating 1 mg/l of chlorine dioxide in an enclosed space through sonication of a 5000 mg/L chlorine dioxide solution. The relative humidity at start should not be higher than 50 %. If the initial humidity of the place is lower than 50%, there is no need to adjust the humidity as the injection of chlorine dioxide gas will simultaneously increase the RH. If the humidity is already above 50%, a dehumidifier will be first used to decrease the RH down to 40-50%.

### Step 3. Liberation of chlorine dioxide gas

The solution of chlorine dioxide as described in step 1, is placed inside a container equipped with an ultrasonic vibrator and is sonicated until the RH of the cabinet reaches 65%. Then, the sonication stops and the concentration of chlorine dioxide gas inside the cabinet is continuously controlled and measured by an in-line flow sensor. If the concentration is within 360±15% ppm, the exposure time is calculated, and the disinfection will be continued for the estimated exposure time. If the concentration is below the target range, the humidity of the room is decreased to an appropriate amount using a dehumidifier. The exact RH decrease should be calculated based on actual conditions. Next, the chlorine dioxide gas is injected via the sonication of the solution. When the RH reaches back 65%, the sonication is stopped, and the concentration of chlorine dioxide is checked again. This process may be repeated a few times to achieve the target concentration (360 ppm).

### Step 4. Determination of the protocol efficacy

To evaluate the efficacy of the proposed method against the target microorganisms, 3 biological indicators are placed inside the cabinet before the disinfection starts. To ensure the method efficacy against a wide range of bacteria, strips coated with geobacillus stearothermophilus is used as the biological indicator. Post disinfection, the strips are removed and incubated at 55 C, and the log reduction is measured after 7 days. A log reduction of 6 is achieved for both readings.

### Example 2. Disinfection of a clean room with no corrosion-sensitive tools

### Step 1. Preparation of chlorine dioxide solution

A solution containing 4000 mg/L of chlorine dioxide is generated from in-situ reaction between concentrated sodium chlorite (25% w/w) and sodium hydrogen sulphate 20% (w/v).

### Step 2. Conditioning of the environment

The relative humidity (RH) of the room is measured before the sonication starts. The desired RH and chlorine dioxide concentration are decided based on customer requirements, formulas, and the disinfection provider experience. Since there are no corrosion-sensitive tools inside the room, higher RHs up to 80% can be used to shorten the exposure time. In this example, the relative humidity will rise by 20 % to obtain 360 ppm chlorine dioxide by sonication of a 4000 mg/L solution in a closed system. So, the initial RH of the room is checked, and if it is less than 60%, the sonication can be immediately started. Otherwise, the RH should be decreased down to 50-60% before the sonication.

### Step 3. Liberation of chlorine dioxide gas

The prepared chlorine dioxide solution is placed inside a container equipped with an ultrasonic vibrator and is sonicated until the RH of the room hits 80%. Then, the sonication is stopped, and the concentration of chlorine dioxide gas inside the room is measured to calculate the exposure time. If the concentration is not lying within the range 360±15% ppm, a dehumidifier will be employed to decrease the humidity to an appropriate amount, which is known by practice, followed by injection of fresh chlorine dioxide gas to increase the concentration as well as the RH back to 80%. Once the concentration is stable around 360 ppm, the exposure time can be calculated.

### Step 4. Determination of the protocol efficacy

The efficacy of the method will be determined using the protocol specified in example 1.

### Example 3. Disinfection of a pass-box in a biological lab with no corrosion-sensitive tools

### Step 1. Preparation of chlorine dioxide solution

A solution containing 3000-4000 mg/L of chlorine dioxide is generated from in-situ reaction between concentrated sodium chlorite (25% w/w) and sodium hydrogen sulphate 20% (w/v).

### Step 2. Conditioning of the environment

The relative humidity (RH) of the pass-box is measured before the sonication starts. Since there are no corrosion-sensitive tools inside the pass-box, higher RHs up to 80% can be used to shorten the exposure time. The initial RH of the room is checked and if it is less than 60%, the sonication can be immediately started. Otherwise, the RH should be decreased down to 50-60% before the sonication.

### Step 3. Liberation of chlorine dioxide gas

The prepared chlorine dioxide solution is placed inside the ultrasonic generator and is sonicated until the RH of the room hits 80%. Then, the sonication is stopped, and the concentration of chlorine dioxide gas inside the pass-box is measured. Since the area to be decontaminated is very small, it is expected that the concentration of chlorine dioxide reaches 360±15% ppm in maximum 1 hour. However, if the concentration is out of the target range, a dehumidifier will be employed to decrease the humidity down to an appropriate amount, which is determined on practice, followed by injection of fresh chlorine dioxide gas to increase the concentration as well as the RH back to 80%. Once the concentration is stable around 360 mg/L, the exposure time can be calculated.

### Step 4. Determination of the protocol efficacy

The efficacy of the method will be determined using the protocol specified in example 1.

## Claims

1. A method for disinfecting a room with chlorine dioxide gas, comprising the steps:
measuring the humidity of the air in the room;
finding the desired initial humidity of the room; preferably between 20-50%;
finding the desired process humidity for disinfection of the room preferably between 20-40% and 50-80% depending on the corrosion sensitivity of the equipment;
reducing the humidity of the room if it is above the desired initial humidity;
generating chlorine dioxide from a liquid in a reaction cell;
releasing said chlorine dioxide from said liquid;
transferring the released chlorine dioxide to the room;
measuring the humidity of the room in real time;
stopping the gas generation at the desired process humidity which was defined before the disinfection;
measuring the concentration chlorine dioxide gas in the room; and
maintaining a constant chlorine dioxide concentration in the air in the room for a defined exposure time,
wherein during the defined exposure time the relative humidity (RH) in the room is kept constant at a predetermined RH between 20% and 80%, **characterized in that,** chlorine dioxide gas is generated from said liquid by an ultrasonic vibrator.

2. Method according to claim 1, **characterized in that,** the method further comprises a step wherein the RH in the room is adjusted between 40% and 50%, prior to the transferring of the released chlorine dioxide gas to the room.

3. Method according to claim 1 or 2, **characterized in that,** the defined exposure time is determined based on the required level of disinfection and the concentration of chlorine dioxide in the room.

4. Method according to claim 1, 2 or 3, **characterized in that,** the liquid is neutralized after the defined exposure time.

5. Method according to any of the previous claims 1 to 4, **characterized in that,** at the end of the defined exposure time, the liquid is mixed with sodium thiosulfate.

6. Method according to any of the previous claims 1 to 5, **characterized in that,** the concentration chlorine dioxide gas in the room is continuously measured by an in-line sensor.

7. Method according to any of the previous claims 1 to 6, **characterized in that,** the liquid comprises 3000-10 000 mg/L of chlorine dioxide.

8. Method according to any of the previous claims 1 to 7, **characterized in that,** the method comprises the step of dehumidifying the room when the RH exceeds a target value.

9. Method according to any of the previous claims 1 to 8, **characterized in that,** the liquid obtained by mixing aqueous solutions of sodium chlorite and sodium bisulfate, preferably the liquid is prepared using concentrated sodium chlorite (25% w/w) and sodium hydrogen sulfate 20% (w/v).

10. Method according to any of the previous claims 1 to 9, **characterized in that,** during the defined exposure time, the concentration of chlorine dioxide in the room is between 300 and 420 ppm.

11. Method according to any of the previous claims 1 to 8, **characterized in that,** the defined exposure time is determined based on the relative humidity (RH) in the room once the concentration of chlorine dioxide in the room is between 300 and 420 ppm.

12. Method according to any of the previous claims 1 to 8, **characterized in that,** 60-100% of the chlorine dioxide is released as a gas from said liquid and 0-40% of the chlorine dioxide is released as droplets with a particle diameter below 40 µm from said liquid.

13. A device for disinfecting a room with chlorine dioxide, comprising:
a RH sensor suitable to measure the relative humidity in the room;
a dehumidifier;
an in-line sensor suitable to measure the chlorine dioxide gas concentration;
a reaction cell for formulating and/or storing chlorine dioxide gas; and
a dispersing unit in fluidic connection with said reaction cell suitable for dispersing the chlorine dioxide gas from said reaction cell in the room,
**characterized in that,** the device further comprises:
an operation panel which is formed of an input portion to input a sterilization condition, and a display portion to display a sterilization status;
an ultrasonic vibrator; and
an electronic control processor, coupled with the in-line sensor and the operation panel, which is configured to control the dehumidifier and the ultrasonic vibrator.

## Patentansprüche

1. Ein Verfahren zum Desinfizieren eines Raumes mit Chlordioxidgas, die folgenden Schritte umfassend:
Messen der Luftfeuchte in dem Raum;
Finden der gewünschten anfänglichen Luftfeuchte des Raumes, vorzugsweise zwischen 20 - 50 %;
Finden der gewünschten Bearbeitungsfeuchte für die Desinfektion des Raumes, vorzugsweise zwischen 20 - 40 % und 50 - 80 %, abhängig von der Korrosionsempfindlichkeit der Ausrüstung;
Reduzieren der Luftfeuchte des Raumes, wenn sie über der gewünschten anfänglichen Luftfeuchte liegt;
Erzeugen von Chlordioxid aus einer Flüssigkeit in einer Reaktionszelle;
Freisetzen des Chlordioxids aus der Flüssigkeit;
Überführen des freigesetzten Chlordioxids in den Raum;
Messen der Luftfeuchte des Raumes in Echtzeit;
Stoppen der Gaserzeugung bei der gewünschten Bearbeitungsfeuchte, die vor der Desinfektion definiert wurde;
Messen der Konzentration des Chlordioxidgases in dem Raum; und
Aufrechterhalten einer konstanten Chlordioxidkonzentration in der Luft in dem Raum für eine definierte Einwirkzeit,
wobei die relative Luftfeuchte (RH) in dem Raum während der definierten Einwirkzeit bei einer festgelegten RH zwischen 20 % und 80 % konstant gehalten wird,
**dadurch gekennzeichnet, dass** Chlordioxidgas aus der Flüssigkeit durch einen Ultraschallvibrator erzeugt wird.

2. Verfahren nach Anspruch 1, **dadurch gekennzeichnet, dass** das Verfahren ferner einen Schritt umfasst, in dem die RH in dem Raum vor dem Überführen des freigesetzten Chlordioxidgases in den Raum auf zwischen 40 % und 50 % eingestellt wird.

3. Verfahren nach Anspruch 1 oder 2, **dadurch gekennzeichnet, dass** die definierte Einwirkzeit basierend auf dem erforderlichen Desinfektionsgrad und der Konzentration des Chlordioxids in dem Raum bestimmt wird.

4. Verfahren nach Anspruch 1, 2 oder 3, **dadurch gekennzeichnet, dass** die Flüssigkeit nach der definierten Einwirkzeit neutralisiert wird.

5. Verfahren nach einem der vorhergehenden Ansprüche 1 bis 4, **dadurch gekennzeichnet, dass** die Flüssigkeit am Ende der definierten Einwirkzeit mit Natriumthiosulfat gemischt wird.

6. Verfahren nach einem der vorhergehenden Ansprüche 1 bis 5, **dadurch gekennzeichnet, dass** die Konzentration des Chlordioxidgases in dem Raum durch einen Inline-Sensor kontinuierlich gemessen wird.

7. Verfahren nach einem der vorhergehenden Ansprüche 1 bis 6, **dadurch gekennzeichnet, dass** die Flüssigkeit 3000 - 10000 mg/l Chlordioxid umfasst.

8. Verfahren nach einem der vorhergehenden Ansprüche 1 bis 7, **dadurch gekennzeichnet, dass** das Verfahren den Schritt des Entfeuchtens des Raumes, wenn die RH einen Sollwert übersteigt, umfasst.

9. Verfahren nach einem der vorhergehenden Ansprüche 1 bis 8, **dadurch gekennzeichnet, dass** die Flüssigkeit durch Mischen wässriger Lösungen von Natriumchlorit und Natriumbisulfat erhalten wird, die Flüssigkeit vorzugsweise unter Verwendung konzentrierten Natriumchlorits (25% w/w) und Natriumhydrogensulfats 20% (w/v) hergestellt wird.

10. Verfahren nach einem der vorhergehenden Ansprüche 1 bis 9, **dadurch gekennzeichnet, dass** die Konzentration des Chlordioxids in dem Raum während der definierten Einwirkzeit zwischen 300 und 420 ppm beträgt.

11. Verfahren nach einem der vorhergehenden Ansprüche 1 bis 8, **dadurch gekennzeichnet, dass** die definierte Einwirkzeit basierend auf der relativen Luftfeuchte (RH) in dem Raum bestimmt wird, sobald die Konzentration des Chlordioxids in dem Raum zwischen 300 und 420 ppm beträgt.

12. Verfahren nach einem der vorhergehenden Ansprüche 1 bis 8, **dadurch gekennzeichnet, dass** 60 - 100 % des Chlordioxids als ein Gas aus der Flüssigkeit freigesetzt werden und 0 - 40 % des Chlordioxids als Tröpfchen mit einem Partikeldurchmesser unter 40 µm aus der Flüssigkeit freigesetzt werden.

13. Eine Vorrichtung zum Desinfizieren eines Raumes mit Chlordioxid, umfassend:
einen RH-Sensor, der geeignet ist, die relative Luftfeuchte in dem Raum zu messen;
einen Entfeuchter;
einen Inline-Sensor, der geeignet ist, die Konzentration des Chlordioxidgases zu messen;
eine Reaktionszelle zum Formulieren und/oder Aufbewahren von Chlordioxidgas; und
eine Dispergiereinheit in Fluidverbindung mit der Reaktionszelle, die geeignet ist, das Chlordioxidgas aus der Reaktionszelle in dem Raum zu verteilen,
**dadurch gekennzeichnet, dass** die Vorrichtung ferner Folgendes umfasst:
eine Bedientafel, die aus einem Eingabeabschnitt zum Eingeben eines Sterilisierungszustandes und einem Anzeigeabschnitt zum Anzeigen eines Sterilisationsstatus gebildet ist;
einen Ultraschallvibrator; und
einen elektronischen Steuerungsprozessor, der mit dem Inline-Sensor und der Bedientafel gekoppelt ist, der dafür konfiguriert ist, den Entfeuchter und den Ultraschallvibrator zu steuern.

## Revendications

1. Procédé permettant de désinfecter une pièce avec du dioxyde de chlore gazeux, comprenant les étapes consistant à :
mesurer l'humidité de l'air dans la pièce ;
trouver l'humidité initiale souhaitée pour la pièce ; de préférence entre 20 et 50 % ;
trouver l'humidité de processus souhaitée pour la désinfection de la pièce
de préférence entre 20 et 40 % et 50 et 80 % en fonction de la sensibilité à la corrosion de l'équipement ;
réduire l'humidité de la pièce si elle est supérieure à l'humidité initiale souhaitée ;
produire du dioxyde de chlore à partir d'un liquide dans une cellule de réaction ;
libérer ledit dioxyde de chlore à partir dudit liquide ;
transférer le dioxyde de chlore libéré dans la pièce ;
mesurer l'humidité de la pièce en temps réel ;
arrêter la production de gaz à l'humidité de processus souhaitée, qui a été définie avant la désinfection ;
mesurer la concentration de dioxyde de chlore gazeux dans la pièce ; et
maintenir une concentration constante de dioxyde de chlore dans l'air dans la pièce pendant une durée d'exposition définie,
dans lequel, pendant le temps d'exposition défini l'humidité relative (HR) dans la pièce est maintenue constante à une HR prédéterminée comprise entre 20 % et 80 %, **caractérisé en ce que,** le dioxyde de chlore gazeux est généré à partir dudit liquide par un vibrateur à ultrasons.

2. Procédé selon la revendication 1, **caractérisé en ce que,** le procédé comprend en outre une étape dans laquelle la HR dans la pièce est ajustée entre 40 et 50 %, avant le transfert du dioxyde de chlore gazeux libéré dans la pièce.

3. Procédé selon la revendication 1 ou 2, **caractérisé en ce que,** le temps d'exposition défini est déterminé en fonction du niveau de désinfection requis et de la concentration de dioxyde de chlore dans la pièce.

4. Procédé selon la revendication 1, 2 ou 3, **caractérisé en ce que,** le liquide est neutralisé après le temps d'exposition défini.

5. Procédé selon l'une quelconque des revendications précédentes 1 à 4, **caractérisé en ce que,** à la fin du temps d'exposition défini, le liquide est mélangé avec du thiosulfate de sodium.

6. Procédé selon l'une quelconque des revendications précédentes 1 à 5, **caractérisé en ce que,** la concentration de dioxyde de chlore gazeux dans la pièce est mesurée en continu par un capteur en ligne.

7. Procédé selon l'une quelconque des revendications précédentes 1 à 6, **caractérisé en ce que,** le liquide comprend 3000 à 10000 mg/L de dioxyde de chlore.

8. Procédé selon l'une quelconque des revendications précédentes 1 à 7, **caractérisé en ce que,** le procédé comprend l'étape de déshumidification de la pièce lorsque la HR dépasse une valeur cible.

9. Procédé selon l'une quelconque des revendications précédentes 1 à 8, **caractérisé en ce que,** le liquide est obtenu par le mélange de solutions aqueuses de chlorite de sodium et de bisulfate de sodium, de préférence le liquide est préparé en utilisant du chlorite de sodium concentré (25 % w/w) et de l'hydrogénosulfate de sodium à 20 % (w/v).

10. Procédé selon l'une quelconque des revendications précédentes 1 à 9, **caractérisé en ce que,** pendant le temps d'exposition défini, la concentration de dioxyde de chlore dans la pièce est comprise entre 300 et 420 ppm.

11. Procédé selon l'une quelconque des revendications précédentes 1 à 8, **caractérisé en ce que,** le temps d'exposition défini est déterminé en fonction de l'humidité relative (HR) dans la pièce une fois que la concentration de dioxyde de chlore dans la pièce est comprise entre 300 et 420 ppm.

12. Procédé selon l'une quelconque des revendications précédentes 1 à 8, **caractérisé en ce que,** 60 à 100 % du dioxyde de chlore est libéré sous forme de gaz à partir dudit liquide et 0 à 40 % du dioxyde de chlore est libéré sous forme de gouttelettes dont le diamètre des particules est inférieur à 40 µm à partir dudit liquide.

13. Dispositif permettant de désinfecter une pièce avec du dioxyde de chlore, comprenant :
un capteur de HR adapté à la mesure de l'humidité relative dans la pièce ;
un déshumidificateur ;
un capteur en ligne permettant de mesurer la concentration de dioxyde de chlore gazeux ;
une cellule de réaction pour la préparation et/ou le stockage de dioxyde de chlore gazeux ; et
une unité de dispersion en liaison fluidique avec ladite cellule de réaction, apte à disperser le dioxyde de chlore gazeux provenant de ladite cellule de réaction dans la pièce,
**caractérisé en ce que,** le dispositif comprend en outre :
un panneau de commande qui est formé d'une partie d'entrée pour entrer une condition de stérilisation, et d'une partie d'affichage pour afficher un état de stérilisation ;
un vibrateur à ultrasons ; et
un processeur de commande électronique, couplé au capteur en ligne et au panneau de commande, qui est configuré pour commander le déshumidificateur et le vibrateur à ultrasons.
